# EUROPEAN PATENT SPECIFICATION

(11) **EP 2 056 820 B1**
(45) Date of publication and mention of the grant of the patent: **06.01.2010**
(21) Application number: 07808509.9
(22) Date of filing: 29.08.2007
(51) Int. Cl.: A61K 31/409, A61K 41/00, A61P 31/10

(54) **A PHARMACEUTICAL COMPOSITION FOR THE TREATMENT OF A FUNGAL SKIN DISORDER AND A METHOD FOR THE PREPARATION THEREOF**
PHARMAZEUTISCHE ZUSAMMENSETZUNG ZUR BEHANDLUNG VON MYKOTISCHEN HAUTERKRANKUNGEN UND VERFAHREN ZU IHRER HERSTELLUNG
COMPOSITION PHARMACEUTIQUE POUR LE TRAITEMENT D'UNE AFFECTION CUTANÉE FONGIQUE ET MÉTHODE DE PRÉPARATION DE LADITE COMPOSITION

(30) Priority: 29.08.2006 NL 1032375
(43) Date of publication of application: 13.05.2009
(73) Proprietor: Stichting voor de Technische Wetenschappen, 3527 JP Utrecht (NL)
(72) Inventor: SMIJS, Geertruida, Maria, Theresia, NL-2318 XP Leiden (NL); SCHUITMAKER, Johannes, NL-2317 ER Leiden (NL)
(74) Representative: Altenburg, Bernardus Stephanus Franciscus
(86) International application number: PCT/NL2007/000211
(87) International publication number: WO 2008/026915

(56) References cited:
- WO-A-2004/069273
- GB-A- 2 415 372
- US-A- 5 053 423
- SMIJS THREES G M ET AL: "Photodynamic treatment of the dermatophyte Trichophyton rubrum and its microconidia with porphyrin photosensitizers." PHOTOCHEMISTRY AND PHOTOBIOLOGY 2004 SEP-OCT, vol. 80, no. 2, September 2004 (2004-09), pages 197-202, XP002481820 ISSN: 0031-8655
- SMIJS THREES G M ET AL: "A novel ex vivo skin model to study the susceptibility of the dermatophyte Trichophyton rubrum to photodynamic treatment in different growth phases." THE JOURNAL OF ANTIMICROBIAL CHEMOTHERAPY MAR 2007, vol. 59, no. 3, March 2007 (2007-03), pages 433-440, XP002481821 ISSN: 0305-7453
- DEMIDOVA TATIANA N ET AL: "Photodynamic inactivation of Bacillus spores, mediated by phenothiazinium dyes." APPLIED AND ENVIRONMENTAL MICROBIOLOGY NOV 2005, vol. 71, no. 11, November 2005 (2005-11), pages 6918-6925, XP002481822 ISSN: 0099-2240
- SMIJS T G M ET AL: "Photodynamic inactivation of the dermatophyte Trichophyton rubrum" PHOTOCHEMISTRY AND PHOTOBIOLOGY, OXFORD, GB, vol. 77, no. 5, 1 May 2003 (2003-05-01), pages 556-560, XP008030728 ISSN: 0031-8655
- LAMBRECHTS S A G ET AL: "Effect of albumin on the photodynamic inactivation of microorganisms by a cationic porphyrin" JOURNAL OF PHOTOCHEMISTRY AND PHOTOBIOLOGY B: BIOLOGY, ELSEVIER SCIENCE S.A., BASEL, CH, vol. 79, no. 1, 4 April 2005 (2005-04-04), pages 51-57, XP004804926 ISSN: 1011-1344
- SMIJS THREES G M ET AL: "Investigation of conditions involved in the susceptibility of the dermatophyte Trichophyton rubrum to photodynamic treatment." THE JOURNAL OF ANTIMICROBIAL CHEMOTHERAPY OCT 2007, vol. 60, no. 4, October 2007 (2007-10), pages 750-759, XP002481823 ISSN: 0305-7453

## Description

The present invention relates to a pharmaceutical composition for photodynamic treatment of a fungal skin disorder.

Several pharmaceutical compositions to treat fungal skin disorders, such as tinea and in particular nail infections are known in the art, e.g. Lamisil™ (terbinafine). They prove to be only moderately effective, and recurrence of the infection is common. This is believed to be caused by spores (conidiae) of the dermatophyte (fungus), the spores apparently not being killed in the treatment. To overcome this problem, efforts have been made to use photodynamic treatment, which should allow a more effective approach as activated oxygen species (such as singlet oxygen) should be less discriminate when it comes to inactivation of the various stages of the dermatophyte. However, while PDT has been shown to be effective against dermatophytes such as *Trichophyton rubrum,* the spores of these dermatophytes appear to survive as they do with topical drugs such as Lamisil™.

The object of the present invention is to provide a pharmaceutical composition capable of inactivating spores (conidiae) and hyphae of dermatophytes.

The pharmaceutical composition according to the present invention as specified in the claims is characterized in that said pharmaceutical composition comprises a photosensitizer and an enhancer, wherein the photosensitizer is a cationic photosensitizer and the enhancer is chosen from the group of i) an acid, and ii) a metal chelating agent.

Surprisingly, the effectiveness of a cationic photosensitizer to inactivate dermatophytes can be extended to spores by incorporation of such an enhancer in the pharmaceutical composition. The pharmaceutical composition may, in addition, comprise a pharmaceutically acceptable carrier or excipient. The cationic photosensitizer in the sense of the present invention is a porphyrin macrocycle photosensitizer having quaternized nitrogen atoms, and is preferably a photosensitizer comprising at least 2, and preferably 3 or more quaternized nitrogen atoms. The presence of side chains bearing one or more negative charges is not excluded, as long as the overall charge of the photosensitizer is positive (with one or more negative counter ions being present). The acid may be any acid, organic or inorganic and including carbonic acid. The pharmaceutical composition is a composition that meets the requirements of a pharmaceutical composition with respect to purity, and is composed of ingredients of pharmaceutical grade. More than one enhancer may be present, preferably of both classes i) and ii). The metal chelating agent is preferably a metal chelating compound capable of chelating Ca²⁺ and Mg²⁺. A suitable example is EDTA or NaEDTA, which may act both as an acid and as a chelating agent. The term "metal chelating compound" encompasses any compound capable of binding metal ions, including for example crown ethers, zeolites, ion-exchangers, proteins (calmoduline) and precipitants (such as negatively charged counter ions). Binding of Ca²⁺ is considered to be of particular advantage. In general the pH of the pharmaceutical composition will be 3 or higher, but below 7, in particular below 6. If the pharmaceutical composition is to be introduced in an aqueous medium before use, for example by dissolving it, said pH is the pH when the resulting pharmaceutical composition is ready for use. The skin to be treated may be subjected to a treatment for removing calcium-ions immediately before treatment by photodynamical therapy (PDT), e.g. using a concentrated EDTA-solution or even ample amounts of demineralised or distilled water. In case of the use of a chelating agent, any excess of the agent may subsequently washed away, again using for example demineralised or distilled water. The invention also comprises porphyrine macrocycles as specified in the claims for use in the treatment of a fungal skin condition using photodynamic treatment with a cationic photosensitizer, and according to a preferred embodiment the treatment involves a step of sequestering calcium, for example as described above or elsewhere in the application. The step of sequestering calcium will be performed before and/or during PDT.

The pharmaceutical composition has a pH of x where 3,5 < x < 6, preferably 4.5 < x < 5.7.

To maintain the pH in this range, especially after contact with the skin, the acid is preferably a weak acid, capable of buffering the pharmaceutical composition in the above ranges. The weak acid has preferably a pKa in the ranges defined above for x.

According to a preferred embodiment, the pharmaceutical composition has an ionic strength of 10 mM or less, more preferably 6 mM or less, most preferably 2 mM or less.

Low ionic strength has been found to have a positive effect on the effectiveness of the photosensitizer in pharmaceutical compositions according to the present invention in the treatment of hyphae.

According to a preferred embodiment, the cationic photosensitizer is a photosensitizer comprising a porphyrin macrocycle, the porphyrin macrocycle comprising at least one quaternized nitrogen atom. More preferably, it has 3 quaternized nitrogen atoms. Currently most preferred is 5,10,15-tris(4-methylpyridinium)-20-phenyl-[21H,23H]-porphine trichloride (Sylsens B).

Important fields of application of the pharmaceutical composition according to the present invention are those wherein the fungal skin disorder is chosen from the group consisting of tinea and onychomycosis.

According to an important embodiment, the pharmaceutical composition is a topical pharmaceutical composition.

Such a topical composition can easily applied on that part of the skin (including nails) that is to be treated. The topical composition is preferably a gel, ointment, cream or any other viscous composition, in particular the aqueous forms thereof. The term topical composition also includes sprays, especially those with volatile solvent such as ethanol or water which allow application to the skin. Non-viscous compositions without a volatile solvent are generally not preferred because they may be more difficult to apply reliably. Water-based topical pharmaceutical compositions, that can be sprayed or rubbed onto the place to be treated, are most preferred.

The present invention also relates to the use of an enhancer for the preparation of a pharmaceutical composition as specified in the claims for the treatment of a fungal infection, wherein the pharmaceutical composition comprises cationic photosensitizer and the enhancer is chosen from the group of i) an acid, and ii) a metal chelating agent.

Preferred embodiments are detailed in the dependent claims, with the advantages as discussed above for the pharmaceutical composition.

The present invention will now be illustrated with reference to the following experiments and the drawing where
Fig. 1 shows the fungicidal effect of Sylsens B at pH 3.5 and pH 7.4;
Fig. 2 similarly shows the fungicidal effect of Sylsens B;
Fig. 3 shows the effect of ionic strength; and
Fig. 4 shows the zeta potential of *T. rubrum* microconidia (filled diamonds) and fungal hyphae (filled squares), measured as a function of the pH. The values given are the mean values for measurements on 3 different microconidia and hyphae isolates including the standard deviation (SD).

### Determination of the Minimal Inhibitory Concentration (MIC)

A polycarbonate membrane filter, 25 mm in diameter and a pore size of 2 µm (Whatman, Breda, The Netherlands) was placed in the central part of a 3 cm culture dish (Greiner, Alphen aan den Rijn, The Netherlands) filled with 5 ml of Malt Extract Agar (MEA). A circular piece of human stratum corneum (SC) with a diameter of 1 cm was subsequently placed on the membrane filter and both were gently brushed with a paintbrush dipped in 70% ethanol. A microconidia suspension isolated from Trichphyton rubrum (CBS nr. 304.60 obtained from Centraal Bureau voor Schimmelcultures, Utrecht, The Netherlands, and prepared as described by Smijs G.M.T. et al (2004) in "Photodynamic treatment of the dermatophyte Trichophyton Rubrum and its microconidia with porphyrin sensitizers. PhotoChemistry and PhotoBiology, 80, 197 - 202") was diluted to 1000 CFU/ml and 15 µl was inoculated on the circular sheet of human SC. The dish was placed in an incubator at 28°C and at 17 hours after spore inoculation photodynamic treatment (PDT) was applied.

At 17 hours after spore inoculation, the membrane filter with SC containing spore inoculates was transported from the MEA dish to a 3 cm culture dish filled with 1035 µl incubation medium. The incubation medium contained 1 ml of distilled water with an adjusted pH (3.5, 4.5, 5.2, or 7.4) and 35 µl of a Sylsens B stock solution (Buchem, Apeldoorn, the Nederland). To optimize the contact of the inoculates with the incubation medium, the membrane filter with the SC was turned upside down during the incubation period of 2 hours. The incubation was performed under continuous shaking conditions. Shortly before the illumination period the membrane filter containing the SC was turned back (surface SC now faces the lamp). In all cases, the illumination time was 1 hour using a red light irradiance of 30 mW/cm². This corresponds to a light dose of 108 J/cm². After the illumination the membrane filter with the SC were transported to a fresh MEA dish, placed in a 28°C incubator and fungal growth was followed for several weeks. Dark controls were included, i.e. the same procedure was carried out except that the inoculated microconidia on human skin were treated with solvent and/or photosensitizer in the dark. In the light controls, the microconidia were treated with solvent (only) in the presence of light. The efficacy of the treatment was expressed as a relative frequency of complete inactivation (killing) of fungal growth detected at day 8 after spore inoculation. This was assessed microscopically with the use of a Zeiss Axiovert 25 (Germany). After day eight no re-growth could be observed by visual inspection in case a complete inactivation of spores and hyphae was obtained after PDT. The treatment effect was followed for a period of several months. Every experiment contained 4 to 6 duplicates of all conditions and the experiments were repeated 3 times at least.

The results are shown in Table 1.

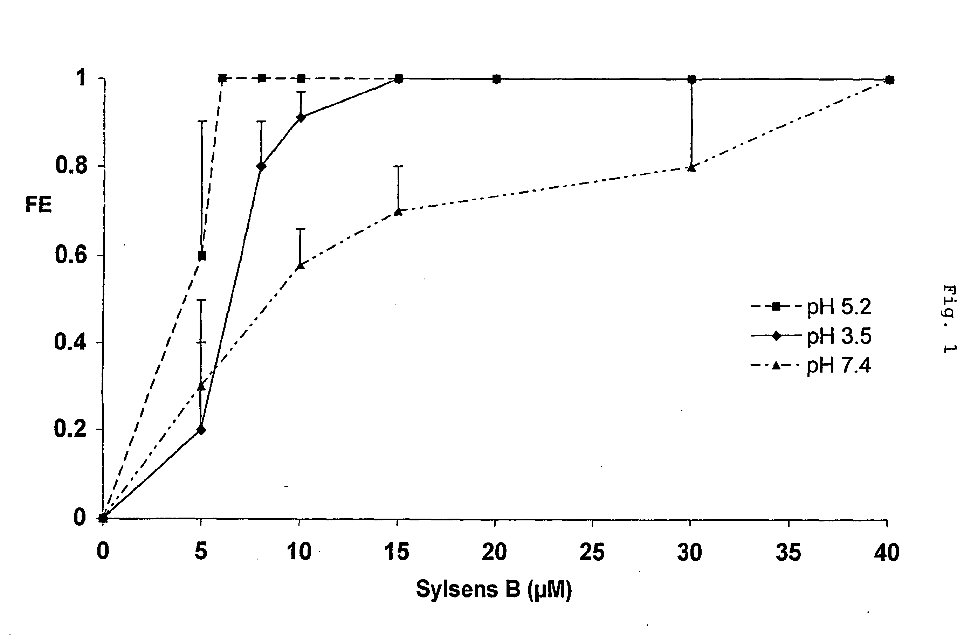

Fig. 1 displays the results of this experiment, for pH 3.5 and 7.4 (fungicidal effect (FE) 8 days after spore inoculation). At an acidic pH, the cationic photosensitizer was significantly more active.

A similar experiment was performed with meso-tetra(N-methyl-4-pyridyl)porphyrin tetrachloride, displaying improved activity at acidic pH, albeit to a lesser extent than Sylsens B (results not shown).

**Table 1**

| µM Sylsens B | pH 3.5 | pH 5.2 | pH 7.4 |
|---|---|---|---|
| 0 | 0 (4) | 0 (4) | 0 (12) |
| 0 (dark control) | 0 (4) | 0 (4) | 0 (6) |
| | | | |
| 0.5 | 0 (6) | 0 (3) | 0 (6) |
| | | | |
| 1 | 0 (6) | 0 (3) | 0 (6) |
| 1 (dark control) | 0 (2) | 0 (2) | 0 (2) |
| | | | |
| 5 | 0.2 (10) | 0.43 (7) | 0.4 (12) |
| | | | |
| 5 (dark control) | 0 (2) | 0 (4) | 0 (6) |
| 6 | 0.5 (2) | 1 (4) | 0.2 (12) |
| 6 (dark control) | | | 0 (6) |
| | | | |
| 7 | | 1 (4) | |
| 8 | 0.75 (4) | 1 (4) | 0.6 (12) |
| 8 (dark control) | | | 0 (6) |
| | | | |
| 9 | | 1 (4) | |
| | | | |
| 10 | 0.8 (10) | 1 (9) | 0.6 (12) |
| 10 (dark control) | 0 (2) | 0 (4) | 0 (6) |
| | | | |
| 15 | 1 (4) | 1 (4) | 0.7 (12) |
| 15 (dark control) | | | 0.5 (6) |
| | | | |
| 20 | 1 (4) | | 0.6 (12) |
| 20 (dark control) | 0 (2) | 0 (2) | 0.3 (6) |
| | | | |
| 30 | 1 (4) | 1 (5) | 0.6 (12) |
| 30 (dark control) | 0.5 (2) | 1 (2) | 0.3 (6) |
| | | | |
| 40 | 1 (4) | | 1 (12) |
| 40 (dark control) | | | 0 (6) |
| | | | |
| 50 | 1 (10) | 1 (3) | 0.9 (12) |
| 50 (dark control) | 0.5 (2) | 1 (2) | 0.5 (6) |
| | | | |
| 65 | 1 (4) | | 1 (12) |
| 65 (dark control) | 1 (2) | | 1 (6) |
| | | | |
| 80 | 1 (8) | 1 (3) | 1 (12) |
| 80 (dark control) | 0.5 (4) | 1 (2) | 1 (6) |
| | | | |
| MIC² | > 10 | 5 - 6 | ≥ 50 |
| | ≤ 15 | | |

| | | | |
|---|---|---|---|
| Incubation: 2 hours in water medium with adjusted pH Illumination: 1 hour, 30 mW/cm² (red light). ¹ The fungicidal effect was determined at 8 days after spore inoculation. ² Minimum Inhibitory Concentration in µM (under the given test circumstances) ³ The dark toxicity is in the same range. ⁴ The fungal growth itself or the germination process (low number of CFU in the light and dark controls) is slow at this pH. | | | |

PDT of *T. rubrum* 17 hours after spore inoculation using the ex-vivo model, different concentrations of Sylsens B and a different pH (water medium). The numbers indicate the relative frequency of the fungicidal effect¹. Numbers between brackets give the number of tests performed. It is obvious from these results that the lowest MIC is found at pH 5.2 indicating that at this pH the sensitizer, upon illumination, is optimal effective as a fungicidal agent.

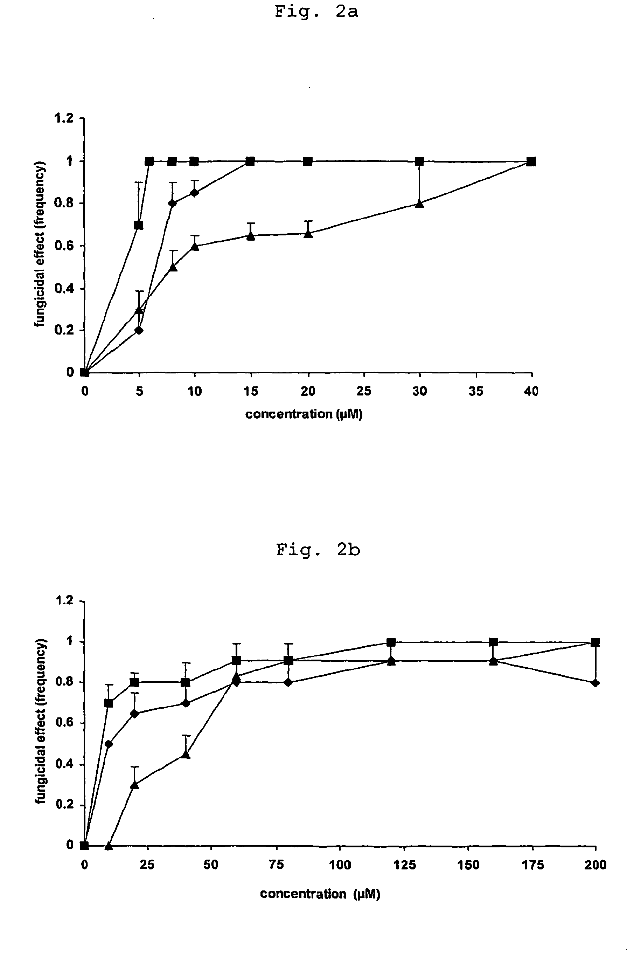

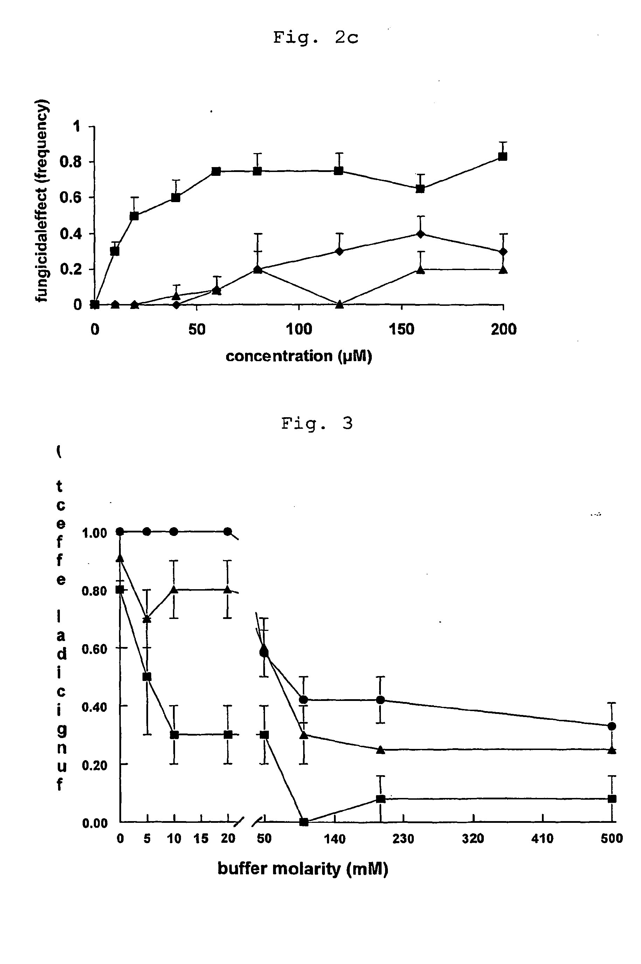

The experiment with Sylsens B was repeated more extensively, for a longer period of time and with more samples. More specifically, the photodynamic efficacy of Sylsens B towards *T. rubrum* was tested at 17 (fig. 2a), 48 (fig. 2b) and 72 hours (fig. 2c) after spore inoculation on human SC, at the following incubation pH values: Filled diamonds: pH 3.5, filled squares: pH 5.2 and filled triangles: pH 7.4. Application of PDT was performed using the *ex-vivo* model with a 2 hours incubation period in distilled water of adjusted indicated pH, followed by 1 hour illumination (30 mW/cm², red light). Values given are the mean values for 5 different experiments and their standard error of mean (SEM). For the 17-hour stage, dark controls displayed 8 to 12 cfu. For the 48-hour and 72 hour stage, dark controls displayed 8 to 12 cfu up to 80 µM Sylsens B and 5 to 8 cfu for higher concentrations. The light controls displayed for all stages 8 to 12 cfu.

### Ionic strength

Reference is made to fig. 3. The photodynamic efficacy of Sylsens B towards *T. rubrum* was tested at 17 (filled circles), 48 (filled triangles) and 72 hours (filled squares) using different molarities of a citric acid/sodium citrate buffer of pH 5.2. Application of PDT was performed using the *ex-vivo model* with a 2 hours incubation period in a citric acid/sodium citrate buffer of pH 5.2 of different molarity, followed by 1-hour illumination (30 mW/cm², red light). The Sylsens B concentration was fixed at 10 µM for PDT application at 17 hours after spore inoculation, 80 µM for application at 48 hours after spore inoculation and 160 µM for application at 72 hours after spore inoculation. Values given are the mean values for 3 different experiments and their SEM.

Both light and dark controls displayed 8 to 12 cfu for all the growth stages.

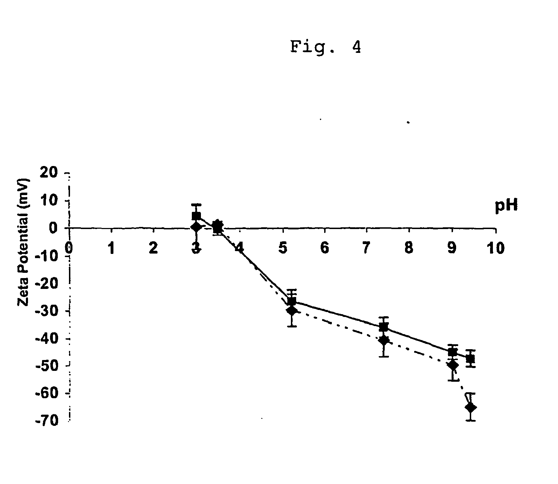

In the above experiments, deuteroporphyrin monomethylether (DP mme), an anionic porphyrin, was used as a control. Being negatively charged, this porphyrin failed to display the observed pH effect. In addition, experiments have been conducted to show that the observed pH effect is not due to different efficacies of Sylsens B at those different pH values. In particular the production of ¹O₂ was substantially constant over the pH range used. In addition, experiments conducted to determine the Zeta potential (using a Zetasizer2000/3000, Malvern Instruments Ltd, Worcs, UK) of microconidia showed that these are negatively charged at a pH above 3.5 (fig. 4), and hence more likely to bind a cationic photosensitizer. Because skin has an isoelectric range centered at a pH of about 5.5, skin will generally be less negatively charged below this pH and hence less likely to bind a cationic photosensitizer. The effect of calcium-ions was investigated by determining the photodynamic effect in the presence of 5 mM CaCl₂ at pH 5.2. This eliminated binding of Sylsens B to the fungus cultivated in suspension. The same effect could be achieved using a positively charged dye, Alcian Blue, which at 160 µM competed with Sylsens B (160 µM) for binding to the fungus, reducing the effectiveness of Sylsens B to inactivate the fungus. So, PDT may even be used to inactivate (kill) the growing fungus if measures are taken to prevent naturally present calcium from interfering with the treatment, in particular by using a chelating compound such as EDTA.

## Claims

1. A pharmaceutical composition for photodynamic treatment of a fungal skin disorder, said pharmaceutical composition comprising a photosensitizer and an enhancer, wherein the photosensitizer comprises a porphyrin macrocycle comprising at least one quaternized nitrogen atom, the enhancer is an acid, and the pharmaceutical composition has a pH of x where 3,5 < x < 6.

2. The pharmaceutical composition according to claim 1, wherein the pharmaceutical composition has a pH of x where 4.5 < x < 5.7.

3. The pharmaceutical composition according to claim 1 or 2, wherein the pharmaceutical composition has an ionic strength of 10 mM or less, more preferably 6 mM or less, most preferably 2 mM or less.

4. The pharmaceutical composition according to any of the preceding claims, wherein the porphyrin macrocycle has 3 quaternized nitrogen atoms.

5. The pharmaceutical composition according to claim 4, wherein the photosensitizer is 5,10,15-tris(4-methylpyridinium)-20-phenyl-[21H,23H]-porphine trichloride.

6. The pharmaceutical composition according to any of the preceding claims, wherein the pharmaceutical composition contains a metal chelating agent.

7. The pharmaceutical composition according to any of the preceding claims, wherein the fungal skin disorder is chosen from the group consisting of tinea and onychomycosis.

8. The pharmaceutical composition according to any of the preceding claims, wherein the pharmaceutical composition is a topical pharmaceutical composition.

9. A method for the preparation of a pharmaceutical composition for the treatment of a fungal infection, wherein the pharmaceutical composition comprises photosensitizer and the preparation involves including an enhancer, wherein the photosensitizer comprises a porphyrin macrocycle comprising at least one quaternized nitrogen atom, the enhancer is an acid, and the pharmaceutical composition has a pH of x where 3,5 < x < 6.

10. The method according to claim 9, wherein a pharmaceutical composition is prepared having a pH of x where 4.5 < x < 5.7.

11. The method according to claim 9 or 10, wherein a pharmaceutical composition is prepared having an ionic strength of 2 mM or less, preferably 0.4 mM or less.

12. The method according to any of the claims 9 to 11, wherein the porphyrin macrocycle has 3 quaternized nitrogen atoms.

13. The method according to claim 13, wherein the photosensitizer is 5,10,15-tris(4-methylpyridinium)-20-phenyl-[21H,23H]-porphine trichloride.

14. The method according to any of the claims 9 to 13, wherein the preparation involves including a metal chelating agent.

15. The method according to any of the claims 9 to 14, wherein the fungal skin disorder is chosen from the group consisting of tinea and onychomycosis.

16. The method according to any of the claims 9 to 15, wherein the pharmaceutical composition is a topical pharmaceutical composition.

## Patentansprüche

1. Eine pharmazeutische Zusammensetzung, für die photodynamische Behandlung von mykotischen Hauterkrankungen, wobei die pharmazeutische Zusammensetzung einen Photosensibilisator und einen Verstärker umfasst, wobei der Photosensibilisator ein cyclisches Porphyrinmakromolekül umfasst, enthaltend zumindest ein quaternäres Stickstoffatom, der Verstärker eine Säure ist und die pharmazeutische Zusammensetzung einen pH-Wert von x hat, wobei gilt: 3,5 < x < 6.

2. Pharmazeutische Zusammensetzung nach Anspruch 1, wobei die pharmazeutische Zusammensetzung einen pH-Wert von x besitzt, wobei gilt: 4,5 < x < 5,7.

3. Pharmazeutische Zusammensetzung nach Anspruch 1 oder 2, wobei die pharmazeutische Zusammensetzung eine Ionenstärke von 10 mmol/l oder weniger aufweist, vorzugsweise von 6 mmol/l oder weniger, weiterhin bevorzugt von 2 mmol/l oder weniger.

4. Pharmazeutische Zusammensetzung nach einem der vorgenannten Ansprüche, wobei das cyclische Porphyrinmakromolekül drei quaternäre Stickstoffatome enthält.

5. Pharmazeutische Zusammensetzung nach Anspruch 4, wobei der Photosensibilisator ein 5,10,15-tris(4-Methylpyridinium)-20-phenyl-[21H,23H]-Porphintrichlorid ist.

6. Pharmazeutische Zusammensetzung nach einem der vorgenannten Ansprüche, wobei die pharmazeutische Zusammensetzung einen mit einem Metall einen Chelatkomplex bildenden Komplexbildner umfasst.

7. Pharmazeutische Zusammensetzung nach einem der vorgenannten Ansprüche, wobei die mykotische Hauterkrankung aus einer Gruppe bestehend aus Tinea und Onychomykose stammt.

8. Pharmazeutische Zusammensetzung nach einem der vorgenannten Ansprüche, wobei die pharmazeutische Zusammensetzung eine Zusammensetzung zur topischen Anwendung ist.

9. Verfahren zur Herstellung einer pharmazeutischen Zusammensetzung für die Behandlung von mykotischen Hauterkrankungen, wobei die pharmazeutische Zusammensetzung einen Photosensibilisator aufweist und die Herstellung das Einbringen eines Verstärkers umfasst, wobei der Photosensibilisator ein cyclisches Porphyrinmakromolekül umfasst, enthaltend zumindest ein quaternäres Stickstoffatom, der Verstärker eine Säure ist und die pharmazeutische Zusammensetzung einen pH-Wert von x hat, wobei gilt: 3,5 < x <6.

10. Verfahren nach Anspruch 9, wobei eine pharmazeutische Zusammensetzung mit einem pH-Wert von x hergestellt wird, wobei gilt: 4,5 < x < 5,7.

11. Verfahren nach Anspruch 9 oder 10, wobei die pharmazeutische Zusammensetzung mit einer Ionenstärke von 2 mmol/l oder weniger hergestellt wir, vorzugsweise von 0,4 mmol/l oder weniger.

12. Verfahren nach einem der Ansprüche 9 bis 11, wobei das cyclische Porphyrinmakromolekül drei quaternäre Stickstoffatome enthält.

13. Verfahren nach Anspruch 12, wobei der Photosensibilisator ein 5,10,15-tris(4-Methylpyridinium)-20-phenyl-[21H,23H]-Porphintrichlorid ist.

14. Verfahren nach einem der Ansprüche 9 bis 13, wobei die die Herstellung das Einbringen eines mit einem Metall einen Chelatkomplex bildenden Komplexbildners umfasst.

15. Verfahren nach einem der Ansprüche 9 bis 14, wobei die mykotische Hauterkrankung aus einer Gruppe bestehend aus Tinea und Onychomykose stammt.

16. Verfahren nach einem der Ansprüche 9 bis 15, wobei die pharmazeutische Zusammensetzung eine Zusammensetzung zur topischen Anwendung ist.

## Revendications

1. Composition pharmaceutique pour le traitement photodynamique d'un affection cutanée fongique, ladite composition pharmaceutique comprenant un photosensibilisateur et un activateur, dans laquelle le photosensibilisateur comprend un macrocycle de porphyrine comportant au moins un atome d'azote quatemisé, l'activateur étant un acide, et la composition pharmaceutique ayant un pH égal à x, où 3,5 < x < 6.

2. Composition pharmaceutique selon la revendication 1, dans laquelle la composition pharmaceutique a un pH égal à x, où 4,5 < x < 5,7.

3. Composition pharmaceutique selon les revendications 1 ou 2, dans laquelle la composition pharmaceutique a une concentration ionique de 10 mM ou moins, préférentiellement de 6 mM ou moins, plus préférentiellement encore de 2 mM ou moins.

4. Composition pharmaceutique selon l'une quelconque des revendications précédentes, dans laquelle le macrocycle de porphyrine comporte 3 atomes d'azote quaternisés.

5. Composition pharmaceutique selon la revendication 4, dans laquelle le photosensibilisateur est du 5,10,15-tris(4-méthylpyridine)-20-phényl, [21H, 23H]-trichlorure de porphine.

6. Composition pharmaceutique selon l'une quelconque des revendications précédentes, dans laquelle la composition pharmaceutique contient un agent chélateur de métaux.

7. Composition pharmaceutique selon l'une quelconque des revendications précédentes, dans laquelle l'affection cutanée fongique est choisie parmi le groupe composé de la teigne et de l'onychomycose.

8. Composition pharmaceutique selon l'une quelconque des revendications précédentes, dans laquelle la composition pharmaceutique est une composition pharmaceutique topique.

9. Procédé pour la préparation d'une composition pharmaceutique pour le traitement d'une infection fongique, dans lequel la composition pharmaceutique comprend un photosensibilisateur et la préparation implique le fait d'inclure un activateur ; procédé dans lequel le photosensibilisateur comprend un macrocycle de porphyrine comprenant au moins un atome d'azote quaternisé, l'activateur étant un acide, et la composition pharmaceutique ayant un pH égal à x, où 3,5 < x < 6.

10. Procédé selon la revendication 9, dans lequel une composition pharmaceutique est préparée en ayant un pH égal à x, où 4,5 < x < 5,7.

11. Procédé selon les revendications 9 ou 10, dans lequel une composition pharmaceutique est préparée en ayant une concentration ionique de 2 mM ou moins, de préférence 0,4 mM ou moins.

12. Procédé selon l'une quelconque des revendications 9 à 11, dans lequel le macrocycle de porphyrine comporte 3 atomes d'azote quaternisés.

13. Procédé selon la revendication 12, dans lequel le photosensibilisateur est du 5,10,15-tris(4-méthylpyridine)-24-phényl-[21H, 23H]-trichlorure de porphine.

14. Procédé selon l'une quelconque des revendications 9 à 13, dans lequel la préparation implique le fait d'inclure un agent chélateur de métaux.

15. Procédé selon l'une quelconque des revendications 9 à 14, dans lequel l'affection cutanée fongique est choisie parmi le groupe composé de la teigne et de l'onychomycose.

16. Procédé selon l'une quelconque des revendications 9 à 15, dans lequel la composition pharmaceutique est une composition pharmaceutique topique.
